Europäisches Patentamt

European Patent Office

Office européen des brevets

(11) Publication number: **0 487 068 A1**

## EUROPEAN PATENT APPLICATION

(21) Application number: **91119791.1**

(22) Date of filing: **19.11.91**

(51) Int. Cl.5: **G01N 33/52**, G01N 31/22

(30) Priority: **21.11.90 JP 319921/90**

(43) Date of publication of application:
**27.05.92 Bulletin 92/22**

(84) Designated Contracting States:
**CH DE FR GB IT LI**

(71) Applicant: **KYOTO DAIICHI KAGAKU CO., LTD.**
**57 Nishiaketa-cho Higashikujo Minami-ku**
**Kyoto-shi Kyoto-fu(JP)**

(72) Inventor: **Mizutani, Satoru**
**29-10, Zendoji-cho, Fukakusa, Fushimi-ku**
**Kyoto-shi, Kyoto-fu(JP)**
Inventor: **Uchigaki, Takatoshi**
**5, Koazahigashitsukurimichi, Oazakamikoma**
**Yamashiro-cho, Soraku-gun, Kyoto-fu(JP)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) Device and method for analyzing liquid sample.

(57) With a device for analyzing a liquid sample comprising a support, a reagent layer provided on the support and a covering which covers at least the reagent layer, is fixed to the support to form a capillary room between the covering and the support and has an opening for supplying a sample and an air outlet opening, a component or components in the liquid sample is accurately and easily analyzed.

## BACKGROUND OF THE INVENTION

### Field of the Invention

The present invention relates to a device and a method for analyzing a liquid sample, in particular, for analyzing a component or components contained in a body liquid including a blood sample such as a whole blood or serum, urine and liquor cerebrospinalis.

### Description of the Related Art

Recently, to quickly and simply analyze a component or components in a liquid sample, in particular, a body liquid such as blood, it is considered useful to use a reagent fixed on a solid support in place of a reagent solution for analysis.

For example, Japanese Patent Publication No. 33800/1974 discloses a water-resistant test film comprising a plastic film carrying a reagent layer which comprises a polymer and a detection system including an oxidase and hydrogen peroxide dispersed in the polymer. In the analysis using this test film, since a generated pigment is measured from a side from which the sample is supplied, the sample such as the whole blood or the serum should be wiped off with absorbent cotton after contacting the sample with a reagent layer for a certain time period. In addition, in order to supply a sufficient amount of oxygen to the reagent layer to develop a color, the test film should be kept in air for a specific time period after wiping off the sample.

Japanese Patent Publication No. 21677/1978 discloses a multi-layer test film comprising a liquid-impermeable transparent support, a reagent layer formed on the support and a spreading layer formed on the reagent layer. When a liquid sample is spotted on the spreading layer, it spreads in the spreading layer and then migrates into the reagent layer. When a pigment is generated through a reaction between a reagent in the reagent layer and a component in the sample, it is measured from the transparent support side, and then the wiping off of the sample is not necessary. However, since the reagent layer is sandwiched between the spreading layer and the support, oxygen in the air hardly reaches the reagent layer. In particular, when the reagent layer contains an oxidase, a sufficient reaction cannot take place due to shortage of oxygen.

To overcome such defects, Japanese Patent Kokai Publication No. 82859/1985 proposes an integral type multilayer chemical analytical element. This analytical element has an oxygen-permeable protein-impermeable light-shielding layer between the spreading layer and the reagent layer, whereby contact of the reagent layer to air is facilitated. However, since a surface of the reagent layer on the support side cannot contact to the air, a sufficient amount of oxygen is not supplied to the reagent layer.

An analytical element disclosed in Japanese Patent Kokai Publication No. 205364/1985 has a hydrophobic porous layer for supplying oxygen between the reagent layer and the support, whereby the reaction in the reagent layer containing the oxidase is greatly facilitated. Since this oxygen-supplying layer has a relatively large thickness for keeping a sufficient amount of oxygen therein, transparency from the support side is not achieved, so that the generated pigment should be measured from the sample-supply side after wiping off the sample.

An analytical element disclosed in Japanese Patent Kokai Publication No. 101757/1988 comprises a porous reagent layer element and a support having perforations. Oxygen is supplied from a surface of the reagent layer on the perforated support side, and the generated pigment is measured from a side opposite to the sample supply side. Therefore, it is not necessary to wipe off the sample. Since it is possible to automatically detect the supply of the sample on the analytical element when a measuring apparatus is used in combination with this analytical element, accuracy of the reaction time measurement and simplicity of measurement are greatly increased. Since the supply of the sample is detected using the transparency of the reagent layer at the same place as the generated pigment is measured, the measurement is influenced by hemocytes when the whole blood is to be analyzed. To remove the influence of the hemocytes, the measurement should be done by a double wavelength method, so that a structure of the measuring apparatus and a correction method become complicated.

## SUMMARY OF THE INVENTION

An object of the present invention is to provide a device and a method for analyzing a liquid sample, which can solve the drawbacks of the above described prior arts.

According to a first aspect of the present invention, there is provided a device for analyzing a liquid sample comprising a support, a reagent layer provided on said support and a covering which covers at least said reagent layer, is fixed to said support to form a capillary room between said covering and said support and has an opening for supplying a sample and an air outlet opening.

According to a second aspect of the present invention, there is provided a method for analyzing a liquid sample using the device for analyzing a liquid sample of the present invention, which method comprises supplying a liquid sample from an opening for supplying a sample in the covering and detecting passage of said liquid sample over said reagent layer to determine the sample supply timing.

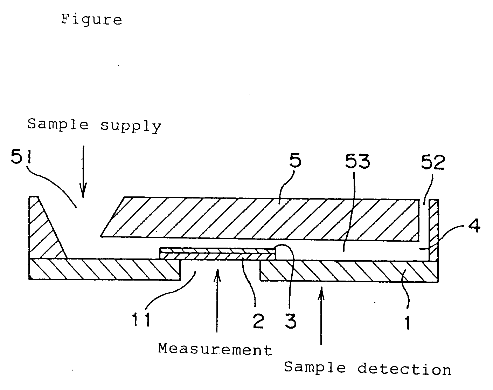

## BRIEF DESCRIPTION OF THE DRAWING

Figure schematically show a cross section of one embodiment of the device for analyzing a liquid sample according to the present invention.

## DETAILED DESCRIPTION OF THE DRAWING

In the device for analyzing a liquid sample according to the present invention, preferably a reagent solution is applied on a transparent film, preferably a transparent porous film and then dried to form a reagent layer, and the transparent film carrying the formed reagent layer is fixed on a support, preferably over a small hole formed in the support. Generally, the hole has a diameter of 2 to 10 mm. Then, the covering is fixed to the support. According to the present invention, the covering is slightly apart from the support and the reagent layer, so that the capillary room is formed between the covering and the reagent layer. A distance between the covering and the support or the reagent layer is not critical. However, if this distance is too large, the sample cannot be quickly spread in the capillary room by capillarity. If this distance is too small, smooth spreading of the liquid sample is prevented by a surface tension and a viscosity of the liquid sample.

Usually, a distance between the covering and the support or the reagent layer is from 0.05 to 0.5 mm, preferably from 0.1 to 0.3 mm.

The transparent film and the covering can be fixed to the support by thermocompression bonding with the use of a thermoplastic resin, or with a double-coated adhesive tape.

The covering has an opening for supplying the liquid sample and an air outlet opening on the respective sides of the reagent layer. These two openings may be formed by perforating the covering, or may be slits formed by cutting out respective parts of the covering.

The transparent film and the reagent layer may have the same structures as those disclosed in Japanese Patent Kokai Publication No. 150751/1990 and EP-A-371 513.

Now, one preferred embodiment of the device for analyzing a liquid sample according to the present invention will be explained by making reference to the accompanying Figure.

The device shown in the Figure comprises a transparent support 1 having a small hole 11, a transparent porous film 2 fixed to the support 1 with covering the hole 11, a reagent layer 3 which is formed on a film surface which does not contact to the support, and a covering 5 which covers at least the reagent layer 3 and is fixed to the support 1 in such way that a capillary room 4 is formed between the covering 5 and the support 1.

The covering has an opening 51 for supplying the liquid sample and an air outlet opening 52. When the liquid sample is supplied from the opening 51, it quickly spreads to a sample detection section 53 with wetting the reagent layer 3 by the capillarity and the gravitational flow effect. Accordingly, when the device of the present invention is set in a suitable measuring apparatus and the liquid sample is analyzed, at substantially the same timing as spotting of the sample (only one second later), the spreading of the sample to the sample detecting section 53 is detected through change of light reflectance at a sample detection point in the Figure. Then, a timer of the measuring apparatus is automatically actuated and, after a specific time period, the measurement is automatically started to give results.

In the present invention, since it is not necessary to make measurement from the reagent side, the reagent layer can be opaque. In addition, since the measurement is not influenced by the hemocytes, the measuring apparatus can be made simple.

## Claims

1. A device for analyzing a liquid sample comprising a support, a reagent layer provided on said support and a covering which covers at least said reagent layer, is fixed to said support to form a capillary room between said covering and said support and has an opening for supplying a sample and an air outlet opening.

2. The device according to claim 1, wherein said reagent layer is formed on a transparent film and said film is fixed to said support.

3. The device according to claim 1, wherein said transparent film is a porous film.

4. The device according to claim 2, wherein said support has a hole and said film covers said hole.

5. The device according to claim 4, wherein said hole has a diameter of 2 to 10 mm.

6. The device according to claim 1, wherein a distance between said reagent layer and said covering is from 0.05 to 0.5 mm.

7. A method for analyzing a liquid sample using a device for analyzing a liquid sample according to claim 1, which method comprises supplying a liquid sample from an opening for supplying a sample in the covering and optically detecting passage of said liquid sample through the capillary room to determine the sample supply timing.

8. The method according to claim 7, wherein passage of said liquid sample over said reagent layer is optically detected.

Figure

Sample supply

51  5  53  52  4  11  2  3  1

Measurement

Sample detection

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 287 883 (MILES INC.)<br>* the whole document *<br>--- | 1-3,6 | G01N33/52<br>G01N31/22 |
| X | EP-A-0 034 049 (EASTMAN KODAK COMPANY)<br>* page 1 - page 8; claims *<br>--- | 1-3 | |
| A,D | EP-A-0 371 513 (KYOTO DAICHI KAGAKU CO., LTD.)<br>* the whole document *<br>--- | 1-3 | |
| A<br><br>D | EP-A-0 256 806 (LIFESCAN, INC.)<br>* abstract; page 24; figures; claims.*<br>& JP-A-63101757<br>--- | 1-3,8 | |
| A | EP-A-0 171 148 (UNILEVER PLC)<br>* page 1 - page 8; claims; figures *<br>--- | 1-3,6,7 | |
| A | EP-A-0 348 006 (PB DIAGNOSTIC SYSTEMS, INC.)<br>* page 1 - page 5 * | 1-3,7 | |
| | ----- | | TECHNICAL FIELDS SEARCHED (Int. Cl.5)<br><br>G01N |

The present search report has been drawn up for all claims

| Place of search | Date of completion of the search | Examiner |
|---|---|---|
| THE HAGUE | 02 MARCH 1992 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document